# EUROPEAN PATENT SPECIFICATION

(11) **EP 1 316 315 B1**
(45) Date of publication and mention of the grant of the patent: **28.12.2011**
(21) Application number: 01958548.8
(22) Date of filing: 28.08.2001
(51) Int. Cl.: A61K 45/00, A61K 38/52, A61P 43/00, A61P 37/08, A61K 38/08

(54) **Serum thymic factor for use in the treatment of oxidative stress in allergy**
Serumthymischer Faktor zur Behandlung von oxidativem Stress bei Allergien
Facteur thymique sérique pour le traitement de maladies de stress oxidatif dans les reactions allergiques

(30) Priority: 28.08.2000 JP 2000256943
(43) Date of publication of application: 04.06.2003
(73) Proprietor: Awaya, Akira, Yokohama-shi, Kanagawa 244 (JP)
(72) Inventor: AWAYA, Akira, Yokohama-shi Kanagawa 244 (JP); ONODERA, Takashi, Ushiku-shi Ibaraki 300-1222 (JP); KAKEGAWA, Tomohito, Chiba-shi Chiba 263-0043 (JP)
(74) Representative: von Kreisler Selting Werner
(86) International application number: PCT/JP2001/007354
(87) International publication number: WO 2002/017965

(56) References cited:
- US-A- 5 464 816
- DATABASE WPI Section Ch, Week 199610 Derwent Publications Ltd., London, GB; Class B04, AN 1996-094122 XP002211536 & JP 08 003057 A (MITSUI TOATSU CHEM INC) 9 January 1996 (1996-01-09)
- IEYOSHI S. ET AL.: 'Studies on mechanism of action of the suppressive effects of facteur thymique serique (FTS) on bleomycin-induced pulmonary fibrosis in mice' THER. RES. vol. 19, no. 10, 1998, pages 3164 - 3166, XP002906749
- VECSEI L. ET AL.: 'The effect of 'facteur thymique serique' (FTS) on catecholamine and serotonin neurotransmission in discrete brain regions of mice' ACTA PHYSIOL. HUNG. vol. 69, no. 1, 1987, pages 129 - 132, XP002906750
- LEVAI J.S. ET AL.: 'The effect of a human plasma thymic factor on human peripheral blood mononuclear cell subpopulations' CLIN. IMMUNOL. IMMUNOPATHOL. vol. 27, no. 3, 1983, pages 433 - 443, XP002906751
- BACH MARIE ANNE ET AL.: 'Effect of long-term treatment with circulating thymic factor on murine lupus' ARTHRITIS RHEUM. vol. 23, no. 12, 1980, pages 1351 - 1358, XP002906752
- ZHAO XUE HUI ET AL.: 'Effects of repeated administrations of facteur thymique serique (FTS) on biochemical changes related to aging in senescence-accelerated mouse (SAM)' JAPAN. J. PHARMACOL. vol. 53, no. 3, 1990, pages 311 - 319, XP002906753

## Description

### Technical Field

The present invention relates to the use of serum thymic factor (FTSnonapeptide) for preparing a medicament for use in treating oxidative stress in allergy and a screening method for searching substances having the activity of FTSnonapeptide.

### Background Art

Many diseases are caused by injuries of each organ and/or tissue, and necrosis and/or apoptosis of cells. Inflammation of a living body elicited by uptake of various foreign substances, their infection, acts as a trigger of these injuries, and further marks the beginning of the repair processes. The foreign substances include chemical substances such as drugs and environmental hormones, viruses, bacteria, protozoan, ultraviolet rays, electromagnetic waves, various organs transplanted from others, blood constituents transfused from others, and further signals from environments and persons which cause psychological stress. Nowadays, from many researches, it is thought that oxidative stresses elicited with radicals, which are so-called ROS (reactive oxygen species) such as NO and active oxygen, are principal cause of bio-injuries.

Living organisms are provided with redox systems against oxidative stresses caused by ROS, and thus homeostasis of living organisms is maintained. Antioxidative enzymes such as catalase, superoxide dismutase (SOD) are present in living organisms. In addition, we take antioxidative vitamines and flavones. A lot of proteins other than the above enzymes, which control redox systems, have been discovered. They include thioredoxins, thioredoxin peroxidases called collectively as peroxiredoxin (including NKEF identified as natural killer cell-enhancing factor), thioredoxin reductases, protein disulfide isomerases, probable protein disulfide isomerases, glutathiones, glutathione peroxidases, glutathione S-transferases, glutathione reductases, hydroperoxideperoxidases, and metalthioneins (Hideyo Sato, Shirou Bannai: Seikagaku 71, 333-337, 1999). Glyceraldehyde 3-phosphate dehydrogenases have been known to be a protein upregulated in ischemic conditions (J. Lab. Clin. Med., 1998 Dec., 132: 456-63). Further, it has been suggested that phosphatidylethanolamine binding protein is a factor involved in resistance to cell death induced by tumor necrosis factor-α (TNF-α) (Elecrtrophoresis, 2002, 21: 660-664).

In the medical field, antiinflammatory effect of steroids was discovered and abuse of steroids, so-called steroid mythology, has been repeated. In the wake of warning to steroids, the research and development of non-steroidal antiinflammatory drugs (NSAIDs) have been extensively performed in the past 40 years, well over aspirin synthesized in the last century. And many NSAIDs have been applied in clinical medicine. Recently, research has been focused on COX-2 inhibitors, and PLA2 inhibitors. Thus, new drugs have been developed and approved.

On the other hand, the research on the mechanisms of induction of expression of proteins involved in afore-mentioned redox regulation that antagonize oxidative stresses which elicit inflammation, alleviate inflammation and thus protect living body has been only started. Therefore, there have no report on drugs that upregulate these proteins, as far as the inventor knows.

### Disclosure of the Invention

Under such circumstance of research, the inventor made a plan to search medical agents having such activities, and searched substances with low molecular weight and weak toxicity. As a results, the inventor found newly that serum thymic factor (FTSnonapeptide, FTS), which has been found to have behaviors and effects for inhibiting injuries and/or destruction of each organ, tissue and cell in monkey, bovine, sheep, dog, cat, rabbit, guinea pig, hamster, rat, mouse, and chicken (References 1-15 and 101-128), have activities and functions leading to express, activating and modulating proteins involved in the afore-mentioned redox regulation, proteins involved in protection of tissue and cell injuries, and proteins involved in supplying systems of energy. Furthermore, the inventor confirmed FTS is a valuable substance as a medicine, accomplished our desired purpose and completed the invention.

That is, the present invention provides the use of serum thymic factor (FTSnonapeptide) for preparing a medicament for treating oxidative stress in allergy.

In addition, the present invention provides an in vitro screening method, which comprises inducing injuries to cells by addition of drugs or oxidative stress, screening for substances that lead to express, activate or modulate proteins selected among intravital redox system regulating proteins, ATP synthetases, glyceraldehyde 3-phosphate dehydrogenases, malate dehydrogenases, heterogenous nuclear ribonucleoprotein Als, 14-3-3 proteins, triosephosphate isomerases, phosphatidylethanolamine binding proteins, sm22-alfa homologs, peptidyl-prolyl cis-trans isomerases, and comparing the determined expression, activatlon or modulation with that obtained with FTSnonapeptide as an index.

### Best Mode for Carrying Out the Invention

The present invention is explained in detail below. The inventor cultivated various tissues and cells described in the references 1-15, induced injuries to the cells by addition of drugs or oxidative stress, and observed how cell death occurred and the dynamics of the proteins that appeared or disappeared. Subsequently, as compared the test group with FTSnonapeptide with the test group without FTSnonapeptide, the inventor found that the proteins which were involved in redox regulation systems, the proteins which were involved in protection of tissue and cell injuries, the proteins which were involved in p energy supplying systems, were lead to express, activated, in some cases modulated, particularly in the group with FTS. In the cells whose mitochondria is injured, where the expression of the proteins which were involved in redox regulation systems was low, it was also found to be normalized in the group with FTS. The change of these proteins can be examined by the measurements of mRNA level of gene encoding the protein, biological activity of the proteins and reactivity to the antibodies against these proteins. The screening methods in terms of these criteria are applicable to exploring other effective drugs. The mRNA level can be analyzed by RT-PCR method, Northern blot method, etc. Further, the inventor compared survival rate of animals in the test group animals with FTS and in the test group animals without FTS, which were two divided groups from animals infected or administered, with viruses, bacteria and drugs such as anti-cancer drugs that were in common foreign substances, and observed the extent of organ injuries histologically and biochemically. As a result, the inventor reconfirmed that survival rate in the test group animals with FTS was high and the extent of the injuries of each organ was weak as reported previously. In parallel with those, it was observed that in terms of histological staining, the proteins in redox regulation systems, the proteins in protection of tissue and cell injuries, the proteins in energy supplying systems, were strongly expressed, and mRNA of these proteins were highly expressed, particularly in the test group animals with FTS. On the other hand, depending on cases, it was found that the expression of those proteins was modulated. Moreover, after non-treated animals including mice were administered with FTS, and various organs were taken off, mRNA level, protein level, biological activity of those proteins and reactivity to the antibodies against those proteins were studied. It was realized that the expression level and the activities of the afore-mentioned proteins were increased or modulated as compared to those of the group without FTS. There are specific peaks of rise and fall or increase and decrease, and transition in the expression of each protein in each cell. Thus, it is necessary to select optimal reaction condition and reaction time for each protein in each cell, in order to search the substances that lead to express, activate or modulate these proteins. Also in animal tests, it is necessary to devise to select optimal administration method, optimal administration schedule, optimal evaluation time for drugs.

A preferable example of drugs whose efficacy was confirmed in these experimental systems is FTSnonapeptide. As described in references 1-15 and 101-128, it is realized that FTS reaches physiologically active concentration in living organisms and exhibits various physiological effects by administering trace quantity of FTS from outside the body. It was frequently happened that regarding many synthetic substances, optimal concentration for their physiological action cannot be achieved in the case they are administered to animals even if they have strong activity in vitro experiments, and desired effects cannot be obtained or side effects are remarkably appeared as they react also with the action sites which elicit other side reactions. In the case of FTS, since it is also a biosubstance in living organisms, its useful pharmacological effects are displayed in the desirable physiological concentration range as it exhibits harmonized effects even if it acts on some action sites.

Cells used in the experiments include epidermic cell, Langerhans' cell, epithelial cell, smooth muscle cell of blood vessel, endothelial cell such as endothelial cell of blood vessel, conbined cells of cardiovascular cell, fibroblast, myocytes, nerve cell, glial cell, other cells of brain, spinal cord, nervous systems, thymus- and spleen-derived immunocyte, each cell of blood, bone marrow cell, bone cell, chondrocyte, synovial cell, osteoblast, osteoclast, interstitial cell of each tissue, pancreatic β cell, cholecystic cell, hepatocyte, hepatic stellate cell, fat cell, mast cell, kidney cell, mesangial cell, respiratory organ system cell such as lung cell, generative cell, spermatozoa, ovum, and urinary organ cell. In addition, tissues used for biochemical or pathological examination were taken and separated from each organ and body fluids of animal individuals.

The injuries to cells and living organisms were elicited by hypoxia and ischemia induced physically or chemically, by the addition or administration of agents such as LPS, TNF, FasL/Fas system proteins, hydrogen oxide, methyl mercury, cadmium, cobalt chloride, cisplatin, adriamycin, 5-fluorouracils, cyclophosphamide, bleomycin, methotrexate, vincristine, cytosine arabinoside, penicillamine, bucillamine, gentamycin, kanamycin, puromycin, cyclosporin, tacrolimus, alloxane, streptozotocin, cerlein, dextransulfate, amiodarone, cephaloridine, and acrylamide by irradiation of ultraviolet rays or X-ray, or further by infection of various viruses, bacteria, fungus and protozoan. In animal experiments, morbid animals, in which injuries were induced artificially by using these agents, by the immunization of brain's, nerves', or muscles' proteins and proteins of living organisms such as collagen or spleen cells were transfused, senescence accelerated mouse (SAM), ally mouse, scid mouse, spontaneously occurred cardiomyopathic hamster which have inherently injuries or diseases, animals that were surgically wounded or burned on surface skin or in organs, and tg mice or ko mice which gene has altered, were prepared.

As the inventor has described on FTS among drug preparations in pharmaceutical compositions of this invention in the patent applications that have been filed so far (References 1-15), it is appropriate that the pharmaceutical compositions of this invention include more improved preparations. As to FTS related substances, the improvement of drug preparations of the proteins such as thymosin-like protein, thymopentin, thymostimulin, and thymomodulin which have been so far considered as the members of thymic factors, is also expected. Each family of proteins whose homologies of amino acid sequence to these peptides and proteins are high or low and medicaments including compounds except for those, can be administered as pharmaceutical compositions in the form of oral or parenteral administration to human and animals.

This invention is described in detail below by showing example.

### Example 1

Mouse insulinoma cell-derived β cell line, Min 6 cells (5x10⁵ /ml) were cultured in Dulbeccco's Modified Eagles medium (DMEM) supplemented with 4500 mg/l of glucose at 37°C under 5% CO₂ + 95% air atomosphere stream. After 48 hours, the culture was transferred to the medium containing 5% FBS, and then 50 ng/ml of TNF-α and 1 ng/ml of FTSnonapeptide or PBS were added to the medium, and the cultivation was started in the 60 mm plates. After 6 hours incubation, the cells were harvested, and washed in PBS at 4 °C. Obtained pellets were dissolved in 0.3 ml of buffers for dissolution [50 mM Tris/HCl (pH8.0), 150 mM NaCl, 0.02% sodium azide, 100 µg/ml PMSF, 1 µg/ml of aprotinin, 1% NP-40]. Proteins dissolved in lysates were centrifuged by 15000x g for 30 min at 4 °C for separation. Protein concentration in the solution was measured by Protein Assay kit (DC Protein Assay; Bio-Rad). Subsequently, 2x SDS gel-loading buffer [boiled 100 mM Tris/HCl (pH6.8), 200 mM dithiothreitol, 4% SDS, 0.2% bromophenol blue, 20% glycerol] was added to the protein solution, followed by boiling, 20 µg of proteins were loaded on 8% SDS-PA gel, and electrophoresed. After electrophoresis, migrated proteins were transferred on PVDF membrane at 15 V for 30 min. Molecular weight of band for the protein, which was specifically observed in the group with FTS, the protein, of which level was increased, or depending on the case, the protein, of which level was modulated, was identified by silver staining or coomassie staining. After ten and more bands of proteins were hydrolyzed and separated by chromatography, some of peptide fragments eluted as many peaks were sequentially sequenced. Homology search of inner sequences showed the results that among those proteins, about 55kD proteins were ATP synthetase β chain and protein disulfide isomerase, about 47kD protein was probable protein disulfide isomerase, 35kD proteins were glyceroaldehyde-3-phosphate-dehydrogenase, malate dehydrogenase and heterogenous nuclear ribonucleoprotein A1, 32kD proteins were 14-3-3 protein ε, ATP synthase γ chain and hemeoxygenase, 28kD proteins were antioxidant enzyme (thioredoxin peroxidase) and triosephosphate isomerase, 26kD proteins were gluthtion-S-transferase and mitochodria thioredoxin dependent peroxidoreductase, 24kD proteins were phosphatidylethanolamine binding protein, sm22-α-homolog and thioredoxin peroxidase 1, 23kD protein was hydroperoxidoglutathion peroxidase, 18kD protein was peptidyl-prolyl-cis-transisomerase, 12kD protein was thioredoxin. Even if plural peaks of peptide fragments were sequenced repeatedly, there were many cases where they were proven to be those of the same proteins. Further, after lysates were electrophoressed, each protein was analyzed by western blotting using antibodies against these proteins that were prepared pro se, or those on sale. As a result, clear bands were detected.

### Example 2

Mouse insulinoma-derived β cell line, Min 6 cells (5x 10⁵ /ml) were cultured in 60 mm plates as likely as described in Example 1. Twelve hours later, many of adhered cells in the group with only TNF-α were rounded, peeled off and floating in the medium. After removal of the floating cells, adhered cells were cetrifuged at 100 rpm for 5 min at 4 °C to harvest. The pellets were resuspended in HMW buffer [100 mM Tris-HCI (pH8.0), 0.5 M NaCI, 25 mM EDTA, 1% SDS] and treated with RNase (10 mg/ml) and Proteinase K. DNA was extracted by phenol-chloroform method, and precipitated with 2.5 fold volume of 100% ethanol. Precipitates were washed with 70% ethanol and dissolved in TE buffer [10 mM Tris-HCI (pH8.0), 2.5 mM EDTA-NaOH (pH8.0)]. In the group with FTS, flaking cells were a little observed, but the numbers were extremely low as compared to those in the group without FTS. One µg of DNA of cells in the group with or without FTS was loaded on 1.5% TAE (Tris-acetate/EDTA) agarose gel, electrophoresed, and stained by ethidium bromide. Then, the electrophoretic pattern was analyzed and compared. In the cells of the group solely added with 50 ng/ml TNF-α, distinctive DNA fragmentation was observed. That is, oligonucleosomal DNA fragments stood in line with ladder formation. On the other hand, in the cells of the group with FTS, distinctive DNA fragmentation was not observed. This fact shows that FTS protects and suppresses strongly apoptosis of Min 6 cells by TNF-α. From this information, it was suggested that the result was based on the action and effects of the proteins whose expression was remarkably induced in the group with FTS in Example 1.

### Example 3

10 pfu of encephalomyocarditis (EMCD) virus, which leads to develop diabetes mellitus and myocarditis, was infected in BALB/c AJcl mice according to the inventor's already reported method [Arch Virol (1996) 141, 73-83]. Two days and a day before the infection, each 50 µg of FTS was administered intraperitoneally in mice. And saline was administered in control group. FTS group didn't develop diabetes mellitus and myocarditis, and control group developed them as likely as already reported. Each organ was taken off, and in situ hybridization was performed. In addition, total RNA was isolated from tissue extracts and RT-PCR was performed. Electrophoresis and Northern blotting were carried out. As to proteins, silver staining or coomassie staining, or western blotting were performed. Each band was detected corresponding to the proteins that regulate intravital redox systems, ATP synthetases, glyceraldehydes-3-phosphate dehydrogenases, malate dehydrogenases, heterogenous nuclear ribonucleoprotein A1s, 14-3-3 proteins, triosephosphate isomerases, phosphatidylethanolamine binding proteins, sm22-alfa homologs, peptidyl-prolyl cis-trans isomerases, as likely as in the results shown in Example 1.

### Example 4

Tubular derived clone cells of transgenic mouse (outer medullary collecting duct; OMCT cells) were cultured in Dulbeccco's Modified Eagles medium (DMEM, supplemented with 4500 mg/l of glucose) at 5% CO₂ + 95% air stream at 37°C to confluence. Cells were transferred to 24 well plates in order to culture in 24 fold areas of medium, and 3 hours later, 0, 0.1, 1, 10 µg/ml of FTS or 0, 0.01, 0.1, 1, 10 µM of thymulin (FTS-Zn) were added and incubated for 6 hours. Then 30 µg/ml of cisplatin, 0.5 µg/ml of methotrexate or 1.5 mM H₂O₂ were added to the cells and the cell were further cultured for 48 hours. The cells were stained with Mayer's hematoxylin solution, and the effects of the drugs on cell proliferation were studied. Addition of 0.1 µg/ml of FTS or 0.01 µM of thymulin to OMCT cells most strongly decreased an inhibitory effect of cell proliferation by cisplatin, methotrexate or H₂O₂. The fact that cell toxicity of three species of compounds whose mechanisms of toxic expression were different were reduced by the same concentration of FTS and thymulin, means that FTS and thymulin have enhancing ability of general resistance of cell to foreign factors that exhibit cell toxicity. By electrophoresis of the cell lysate as likely as in Example 1, proteins were separated and sequenced. As a result, similar results were obtained as those in Example 1.

### Example 5

Five weeks old male ICR mice were divided to 7 groups, and the 1st group were subcutaneously administered with saline daily as a control group. The 2nd and 3rd group were respectively administered with 0.5 mg/kg or 1.0 mg/kg of FTS subcutaneously only once a day. After 24 hours, they were slaughtered and some of organs were minced. Content of metallothionein (MT) of which assay system was present were measured in those organs. The 4th and 5th group were respectively administered with 0.5 mg/kg or 1.0 mg/kg of FTS subcutaneously once a day for three days. Twenty four hours after the last administration, MT content was assayed similarly. The 6th and 7th group were respectively administered with 0.5 mg/kg or 1.0 mg/kg of FTS subcutaneously once a day for five days. Twenty four hours after the last administration, MT content was assayed similarly. MT contents of liver, thymus and spleen were increased significantly in FTS-administered groups as compared with those of control. As to liver, one or three administration of both doses of FTS increased MT content 1.7-2.1 fold. As to thymus, one, three or five administration of FTS increased MT content 3.7-5.7 fold. As to spleen, once, three or five administration of FTS increased MT content 2.0-2.8 fold. MT contents of each organ didn't decrease to under the lower limit of magnification on 6 days after FTS administration.

### Example 6

Eleven weeks old female BALB/c mice were administered with 10 µg of FTS dissolved in 50 µl of saline, and control group of mice were intraperitoneally administered with 50 µl of saline. After 20,60 or 180 minutes, thymus was taken off and total RNA was extracted. The expression of genes was analyzed semi-quantitatively by RT-PCR. As the results, expression of thioredoxin mRNA and glutathione-S-transferase mRNA at 20, 60, 180 minutes after the injection, and thioredoxin peroxidase mRNA at 180 minutes in FTS-treated group was shown to be increased as compared with those of control group. Significant difference of the expression of actin mRNA was not seen between FTS-treated mice and control mice.

### Industrial Applicability

Drug therapy based on symptomatic treatment has been carried out extensively to repair many diseases and injuries based on damages and destruction of organs, tissues and cells. On the other hand, wound healing-stimulation drugs have been proposed that promote repairing activity and regenerating activity of tissues and cells. However, there is no other therapy that surpasses organ transplantation to treat serious organ injuries. In order to protect urgent, inflammatory, immunological, vascular injuries such as myocardial infarction, cerebral infarction, cerebral ishemia, cerebral hemorrhage, post ischemic reperfusion injuries, severe infection, septic shock, serious trauma, burns, hemorrhagic shock, multiple organ failure, fulminating hepatitis, respiratory pause, anaphylactic shock, and graft versus host reaction disease (GVHD) ... at transplantation of kidney, lung, liver, pancreas, heart, skin, cornea, cerebral cells and nervous tissues , and so as to alleviate the injuries as far as possible, there are no ways other than mobilizing all the enzyme proteins defending oxidative stress, the proteins involved in protection and regeneration of tissue and cell injuries, and the proteins involved in energy providing systems, and cytokines, and growth and/or differentiation factors, all provided in living organisms. On the other hand, substances to prevent and obstruct daily, slowly-progressed lifestyle-related diseases (diabetes mellitus, lipid metabolizing disease, arteriosclerosis, various kinds of granulocyte-inducing diseases, stresses occurred in the continuous sympathetic nerve-dominant life, ishemic coronary disease, cerebro and neuro degenerative disease, rheumatoid arthritis, various kinds of renal diseases, various kinds of hepatic diseases, ulcerative colitis, various kinds of autoimmune diseases, cancers ) and food allergy, further demyelinating diseases such as multiple sclerosis, myasthenia gravis, amyotrophic lateral sclerosis, muscular dystrophy, multiple neuritis, SLE, scleroderma, Behçet's were also the enzyme proteins defending oxidative stress, the proteins involved in protection and regeneration of tissue and cell injuries, and the proteins involved in energy providing systems, and cytokines, and growth and differentiation factors, all provided in living organisms. Peptides such as FTS originally present in living organisms are thought to be situated as control towers or mediators that have a function inducing such defending proteins.

Research, which explores substances regulating oxidative stress such as FTS, is exceedingly important, as drugs that can be administered externally. Substances selected by the screening methods disclosed by this invention are accepted as drugs, which acts similarly as FTS. They will be worthy of being examined to develop as drugs similar or comparable to a drug such as FTS that is a biogenic substance, and exhibits clear medicinal virtues by oral or parenteral administration in living organisms.

### References

1. Tokkaihei 08-119876,
2. Tokkaihei 08-092122,
3. Tokkaihei 08-027019,
4. Tokkaihei 08-003059,
5. Tokkaihei 08-003057,
6. Tokkaihei 06-192120,
7. Tokkaihei 06-135849,
8. Japanese Patent 2709839,
9. Japanese Patent2655357,
10. Japanese Patent2655343,
11. Tokkaihei 01-316328
12. Tokkaihei 01-230529,
13. Japanese Patent3159441 (Tokkaihei 3-204819)
14. Tokuganshou 62-291815
15. Tokkouhei 3-71413 (Tokkaishou 58-52225) (USP 5,112,810)
101. Effects of Repeated Administrations of Facteur Thymique Serique (FTS) on Biochemical Changes Related to Aging in Senescence-Accelerated Mouse (SAM). Xue-Hui Zhao, Akira Awaya, Yukiko Tokumitsu and Yasuyuki Nomura et al.: Japan. J. Pharmacol. 53, 311-319 (1990).
102. Radioprotective Effects of Serum Thymic Factor in Mice. Hisashi Kobayashi, Hayao Abe, Tsutomu Ueyama, Akira Awaya and Mikio Shikita: Radiation Research 129, 351-356 (1992).
103. Serum thymic factor as a radioprotective agent promoting survival after X-irradiation. H. Kobayashi, H. Abe, A. Awaya, H. Inano and M. Shikita: Experientia 46, 484-486, 1990.
104. A Thymic Hormone Protects Mice from Enteropathy during Acute Graft-versus-Host Disease. Kyoko Inagaki-Ohara, Tetsu Sakai, Goyo Koya, Akira Awaya and Yasunobu Yoshikai: Microbiol. Immunol., 41 (11) 883-889, 1997.
105. Augmentation with Serum Thymic Factor of Suppressive Effects on Retroviral Tumor Development in Hosts Immune to V-Onc Product. Motohiro Miyauchi, Nobuko Koshikawa, Akira Awaya and Koshi Maruyama et al.: Leukemia 11 suppl. 3, 213-215, April 1997.
106. Effects of a Nonapeptide Thymic Hormone on Intestinal Intraepithelial Lymphocytes in Mice Following Administraion of 5-Fluorouracil. Kyoko Inagaki-Ohara, Noritada Kobayashi, Akira Awaya and Yasunobu Yoshikai et al.: Cellular Immunology 171, 30-40 (1996).
107. In vivo administration of serum thymic factor (FTS) prevents EMC-D virus-induced diabetes and myocarditis in BALB/cAJcl mice. M. Mizutani, M. El-Fotoh, A. Awaya, Y. Matsumoto and T.Onodera et al.: Arch Virol (1996) 141: 73-83.
108. FTS reduces bleomycin-induced cytokine and chemokine production and inhibitspulmonary fibrosis in mice. S. Yara, K. Kawakami, A. Awaya and A. Saito et al.: Clin.Exp.Immunol. 2001; 124: 77-85.
109. Activation of Canine Monocytes and Polymorphonuclear Cells by Serum Thymic Factor (FTS) in vivo. Toshifumi Kosaka, Masayoshi Yukawa, Akira Awaya, Takashi Onodera et al.: J. Vet. Med. Sci. 58 (4): 323-327, 1996.
110. Effect of Administration of Serum Thymic Factor (FTS) in Calves and Rabbits Infected with Bovine Immunodeficiency-Like Virus. Nobuaki Hirai, Hiroyuki Furuyama, Akira Awaya and Misao Onuma: J. Vet. Med. Sci. 57 (2): 307-310, 1995.
111. Resistance to Pseudorabies Virus with Enhanced Interferon Production and Natural Killer Cell Activity in Mice Treated with Serum Thymic Factor. Takashi Onodera, Kazuhiro Yoshihara, Akira Awaya, Masayoshi Yukawa et al.: Microbiol. Immunol., 38 (1), 47-53, 1994.
112. Protective effects of serum thymic factor to Leptospira Interrogans serovar Copenhageni infection in Mongolian gerbils. M. Yukawa, K. Mochizuki, T. Kosaka, A. Awaya, T. Onodera et al.: Veterinary Microbiology 41 (1994) 99-106.
113. Thymic atrophy in type 2 reovirus infected mice: Immunosuppression and effects of thymic hormone. Thymic atrophy caused by reo-2. Takashi Onodera, Toshiaki Taniguchi, Akira Awaya and Toshiharu Hayashi et al.: Thymus 18, 95-109, 1991.
114. Anti-thyroglobulin antibodies induced with recombinant reovirus infection in BALB/c mice. T.Onodera and A.Awaya: Immunology 1990, 71, 581-585.
115. The distribution and structure of FTS immunoreactive cells in the thymus of the mouse. Shigeko Fujiwara, Hisashi Kobayashi, Akira Awaya: Arch. Histol. Cytol. Vol. 51, No. 5 (1988) p.467-472.
116. Enhanced Interferon Production and Natural Killer Cell Activity in Mice Treated with Serum Thymic Factor. T. Onodera, A. Awaya, M. Mizutani and M. Yukawa: Bull. Soc. Fr.-Jpn. Sci. Vet. 6 (2) 68-76, 1995.
117. Vascular and stromal changes in irradiated and recovering rat thymus. Y. Wang, N. Tokuda, M. Tamechika, N. Hashimoto, A. Awaya, T. Sawada and T. Fukumoto et al.: Histol Histopathol (1999) 14: 791-796.
118. Mechanisms of prevention of induction of diabetes melitus by serum thymic factor (FTS). Toshikazu Yamanouchi,Hitoshi Moromizato, Kumiko Irie, Akira Awaya, Hideo Miyashita, leo Akaoka et al.: Proceedings of The Japan Society of Clinical Biochemistry and Metabolism, Vol. 30, 124-125, 1994.
119. Inhibitory effects of FTS on the mouse metastatic lung cancer and their prolongation by using liposomes.: Shuji Kojima, Takaharu Nomura, Kazuhiko Kubota, Akira Awaya, Tsutomu Yuda, Kazuo Maruyama, Motoharu Iwatsuru; Drug Delivery System: 8, 39-44 (1993).
120. Therapeutic effect of FTS (facteur thymique serique) on experimental pancreatitis.: Kenji Okubo, Kyoko Miyasaka, Aturou Jimi, Akihiro Funakoshi, Akira Awaya et al.; Gendai Iryou (Modern medicine) Vol. 25 (addition III): 2947, 1993.
121. Suppressive effects of FTS on alloxan-induced diabetes of mice and their prolongation by using liposomes.: Shuji Kojima, Akira Awaya, Kazuo Maruyama, Motoharu Iwatsuru; Drug Delivery System 7, 357-361 (1992).
122. Prevention of tacrolimus induced-renal disorders by serum thymic factor (FTS): Atsuko Nakashima, Hitoshi Tada, Kazuyuki Inoue, Akiko Fujisaki, Kunihiko Ito, Toshio Suzuki, Seiko Yanagihara, Akira Awaya; Farumashia 35 (10) 1090, (1999).
123. Administration of serum thymic factor (FTS) on cardiomyopathic hamster. Mitsutoshi Kato, Masahito Tsuchiya, Nobuakira Takeda, Seibu Mochizuki, Makoto Nagano, Akira Awaya et al.: Journal of Molecular and Cellular Cardiology Vol. 28, No. 5, A66 (260), May 1996.
124. The effects of serum thymic factor(FTS)on cardiomyopathic hamster. Mitsutoshi Kato, Masahito Tsuchiya, Nobuakira Takeda, Seibu Mochizuki, Makoto Nagano, Akira Awaya et al.: Journal of Molecular and Cellular Cardiology Vol. 27, No. 11, A534 (131), November 1995.
125. Prevention of diabetes by thymic hormon in alloxane-treated rats. Toshikazu Yamanouchi, Hitoshi Moromizato, Shuji Kojima, Takaomi Shinohara, Hideo Miyashita, leo Akaoka et al.: European J Pharmacology 257 (1994) 39-46.
126. Reovirus type 2 induced diabetes prevented with immunosuppression and thymic hormone. Onodera T., Taniguchi T., Yoshihara K., Shimizu S., Sato M., Hayashi T.: Diabetologia 33; 192-196 (1990).
127. Suppression of acute experimental allergic encephalomyelitis by synthetic serum thymic factor. Kato S. Nakamura H.: Acta Neuropathol., 1988: 75; 337-344.
128. Intensive suppression of EAE by serum thymic factor and therapeutic implication for multiple sclerosis. Nagai Y., Osanai T., Sakakibara K.: Japanese Journal of Experimental Medicine, 1982: 52; 213-219.

## Claims

1. Use of serum thymic factor (FTSnonapeptide) for preparing a medicament for treating oxidative stress in allergy.

2. An in vitro screening method, which comprises inducing injuries to cells by addition of drugs or oxidative stress, screening for substances that lead to express, activate or modulate proteins selected among intravital redox system regulating proteins, ATP synthetases, glyceraldehyde 3-phosphate dehydrogenases, malate dehydrogenases, heterogenous nuclear ribonucleoprotein Als, 14-3-3 proteins, triosephosphate isomerases, phosphatidylethanolamine binding proteins, sm22-alfa homologs, peptidyl-prolyl cis-trans isomerases, and comparing the determined expression, activation or modulation with that obtained with FTSnonapeptide as an index.

3. The screening method of claim 2, wherein the inducing of injuries is effected by the addition or administration of agents selected from LPS, TNF, FasL/Fas system proteins, hydrogen oxide, methyl mercury, cadmium, cobalt chloride, cisplatin, adriamycin, 5-fluorouracils, cyclophosphamide, bleomycin, methotrexate, vincristine, cytosine arabinoside, penicillamine, bucillamine, gentamycin, kanamycin, puromycin, cyclosporin, tacrolimus, alloxane, streptozotocin, cerlein, dextransulfate, amiodarone, cephaloridine, and acrylamide, by irradiation of ultraviolet rays or X-ray, or by infection of various viruses, bacteria, fungus, and protozoan.

## Patentansprüche

1. Verwendung von Thymus-Serum-Faktor (FTS-Nonapeptid) zur Herstellung eines Medikaments zur Behandlung von oxidativem Stress bei einer Allergie.

2. In-vitro-Screening-Verfahren, das Folgendes umfasst: das Induzieren von Verletzungen bei Zellen durch Zugabe von Wirkstoffen oder durch oxidativen Stress, das Screening nach Substanzen, die dazu führen, Proteine zu exprimieren, zu aktivieren oder zu modulieren, die aus intravitalen Redoxsystem-regulierenden Proteinen, ATP-Synthetasen, Glyceraldehyd-3-phosphat-Dehydrogenasen, Malat-Dehydrogenasen, heterogenem Kernribonucleoprotein Als, 14-3-3-Proteinen, Triosephosphat-Isomerasen, Phosphatidylethanolamin-Bindungsproteinen, sm22-α-Homologen und Peptidyl-Prolyl-cis-trans-Isomerasen ausgewählt sind, und das Vergleichen der bestimmten Expression, Aktivierung oder Modulation mit derjenigen, die man mit FTS-Nonapeptid erhält, als Index.

3. Screening-Verfahren gemäß Anspruch 2, wobei das Induzieren von Verletzungen durch die Zugabe oder Verabreichung von Mitteln, die aus LPS, TNF, Proteinen des FasL/Fas-Systems, Wasserstoffperoxid, Methylquecksilber, Cadmium, Cobaltchlorid, Cisplatin, Adriamycin, 5-Fluoruracilen, Cyclophosphamid, Bleomycin, Methotrexat, Vincristin, Cytosinarabinosid, Penicillamin, Bucillamin, Gentamicin, Kanamycin, Puromycin, Cyclosporin, Tacrolimus, Alloxan, Streptozotocin, Caerulein, Dextransulfat, Amiodaron, Cephalodirin und Acrylamid ausgewählt sind, durch Bestrahlung mit ultravioletten Strahlen oder Röntgenstrahlen oder durch Infektion mit verschiedenen Viren, Bakterien, Pilzen und Protozoen bewirkt wird.

## Revendications

1. Utilisation du facteur thymique sérique (FTSnonapeptide) pour la préparation d'un médicament destiné au traitement du stress oxydatif dans le cas d'une allergie.

2. Procédé de sélection in vitro, qui comprend l'induction de lésions dans les cellules par addition de médicaments ou stress oxydatif, la sélection de substances qui conduisent à l'expression, l'activation ou la modulation de protéines choisies parmi les protéines de régulation du système redox intravital, les ATP synthétases, les glycéraldéhyde-3-phosphate-déshydrogénases, les malate-déshydrogénases, la ribonucléoprotéine nucléaire hétérogène Als, les protéines 14-3-3, les triose-phosphate-isomérases, les protéines de liaison de la phosphatidyléthanolamine, les homologues de sm22-alfa, les peptidylpropyl-cis-trans-isomérases, et la comparaison de l'expression, de l'activation ou de la modulation déterminée avec celle obtenue à l'aide du FTSnonapeptide sous forme d'index.

3. Procédé de sélection selon la revendication 2, dans lequel l'induction des lésions s'effectue par l'addition ou l'administration d'agents choisis parmi LPS, TNF, les protéines du système FasL/Fas, l'oxyde d'hydrogène, le méthylmercure, le cadmium, le chlorure de cobalt, le cisplatine, l'adriamycine, les 5-fluorouraciles, le cyclophosphamide, la bléomycine, le méthotrexate, la vincristine, la cytosine arabinoside, la pénicillamine, la bucillamine, la gentamycine, la kanamycine, la puromycine, la cyclosporine, le tacrolimus, l'alloxane, la streptozotocine, la cerléine, le dextran sulfate, l'amiodarone, la céphaloridine et l'acrylamide, par irradiation de rayons ultraviolets ou de rayons X, ou par l'infection de différents virus, bactéries, champignons et protozoaires.
